# EUROPÄISCHE PATENTANMELDUNG

(11) **EP 4 074 350 A1**
(43) Veröffentlichungstag der Anmeldung: **19.10.2022**
(21) Anmeldenummer: 22165353.8
(22) Anmeldetag: 30.03.2022
(51) Int. Cl.: A61M 1/00

(54) **CHIRURGISCHES ABSAUGSYSTEM**

(30) Priorität: 13.04.2021 DE 102021109211
(71) Anmelder: Cheufou, Danjouma Housmanou, 97082 Würzburg (DE)
(72) Erfinder: Cheufou, Danjouma Housmanou, 97082 Würzburg (DE)
(74) Vertreter: advotec.

(57) **Zusammenfassung**

Die Erfindung betrifft eine Absaugvorrichtung (6) zur Durchführung eines chirurgischen Eingriffs bei einem Menschen oder einem Tier, umfassend: ein Saugrohr (2), wobei das Saugrohr (2) zum Abführen von angesaugtem Blut und angesaugten Sekreten vorgesehen ist, eine vordere Öffnung (5) des Saugrohrs (2) zur Aufnahme von angesaugtem Blut und angesaugten Sekreten, wobei ein Schwamm (1) vor der Öffnung (5) angeordnet ist.

## Beschreibung

### GEBIET DER ERFINDUNG

Die vorliegende Erfindung betrifft eine Vorrichtung zum Absaugen von Blut und Sekreten aus einer Operationswunde eines Menschen oder eines Tieres.

### HINTERGRUND DER ERFINDUNG

Im Stand der Technik sind Absaugsysteme bekannt, die ein Absaugen von Blut und Sekreten aus einer Operationswunde erlauben. Das Dokument DE 10 2016 125 556 A1 beschreibt eine Saugerspitze zum Absaugen von Blut aus einem Operationsfeld. Die DE 10 2008 038 908 A1 offenbart eine Saugvorrichtung zum Absaugen von Flüssigkeit. Das Dokument DE 10249674 B4 beschreibt ein Operationsinstrument zum Schneiden, Abtragen und Absaugen von Material in einem Operationsgebiet.

### ZUSAMMENFASSUNG DER ERFINDUNG

Das Absaugen von Blut und Sekreten aus einem Operationsgebiet sollte möglichst schnell und umfassend erfolgen, um die Operationszeit möglichst gering zu halten. Es ist daher eine Absaugvorrichtung für das Operationsgebiet erforderlich, das großflächig und effektiv auch größere Mengen an Blut und Sekreten aus dem Operationsgebiet entfernen kann.

Eine Aufgabe ist daher, eine Vorrichtung zur Verfügung zu stellen, durch die das Entfernen von Blut und Sekreten aus einem Operationsgebiet schnell und umfassend erfolgen kann.

Als erste Ausführungsform der Erfindung wird eine Absaugvorrichtung zur Durchführung eines chirurgischen Eingriffs bei einem Menschen oder einem Tier zur Verfügung gestellt, umfassend: ein Saugrohr, wobei das Saugrohr zum Abführen von angesaugtem Blut und Sekreten vorgesehen ist, und eine vordere Öffnung des Saugrohrs zur Aufnahme von angesaugtem Blut und Sekreten, wobei ein Schwamm vor der Öffnung angeordnet ist.

Beispielhafte Ausführungsformen werden in den abhängigen Ansprüchen beschrieben.

Gemäß einer beispielhaften Ausführungsform der Erfindung wird eine Absaugvorrichtung zur Verfügung gestellt, wobei die Absaugvorrichtung eine seitliche Öffnung zur Aufnahme von angesaugtem Blut und Sekreten aufweist.

Mit einer oder mehreren seitlichen Öffnungen des Absaugrohrs kann nicht nur punktuell, sondern über eine Fläche eine Absaugung von Blut und Sekreten vorgenommen werden.

Gemäß einer beispielhaften Ausführungsform der Erfindung wird eine Absaugvorrichtung zur Verfügung gestellt, wobei vor der seitlichen Öffnung der Schwamm angeordnet ist.

Der Schwamm sammelt das angesaugte Blut und die Sekrete auf, wodurch das Blut und die Sekrete aus der Operationswunde entfernt werden können.

Als eine Idee der Erfindung kann angesehen werden, eine Absaugvorrichtung für eine chirurgische Behandlung zur Verfügung zu stellen, die möglichst umfassend Blut und Sekrete aus der Operationsöffnung entfernen kann. Hierzu weist die Absaugvorrichtung eine vordere Absaugöffnung und in einer alternativen Ausführungsform seitliche Absaugöffnungen auf. Sämtliche Absaugöffnungen sind von einem Schwamm ummantelt, sodass angesaugtes Blut und Sekrete in dem Schwamm aufgenommen werden können und damit aus der Operationsöffnung entfernt werden können. Vorteilhafterweise wird durch den Schwamm ein Trockenlegen der Operationswunde erreicht, wodurch der chirurgische Eingriff erleichtert wird.

Die einzelnen Merkmale können selbstverständlich auch untereinander kombiniert werden, wodurch sich zum Teil auch vorteilhafte Wirkungen einstellen können, die über die Summe der Einzelwirkungen hinausgehen.

### KURZE BESCHREIBUNG DER ZEICHNUNGEN

Weitere Einzelheiten und Vorteile der Erfindung werden anhand der in den Zeichnungen dargestellten Ausführungsbeispiele deutlich. Es zeigen:
- Fig. 1: eine erste Ausführungsform einer erfindungsgemäßen chirurgischen Absaugvorrichtung 6 und
- Fig. 2: eine zweite Ausführungsform einer Absaugvorrichtung 6 mit, neben der vorderen Absaugöffnung 5, seitlichen Absaugöffnungen 7.

### DETAILLIERTE BESCHREIBUNG BEISPIELHAFTER AUSFÜHRUNGSFORMEN

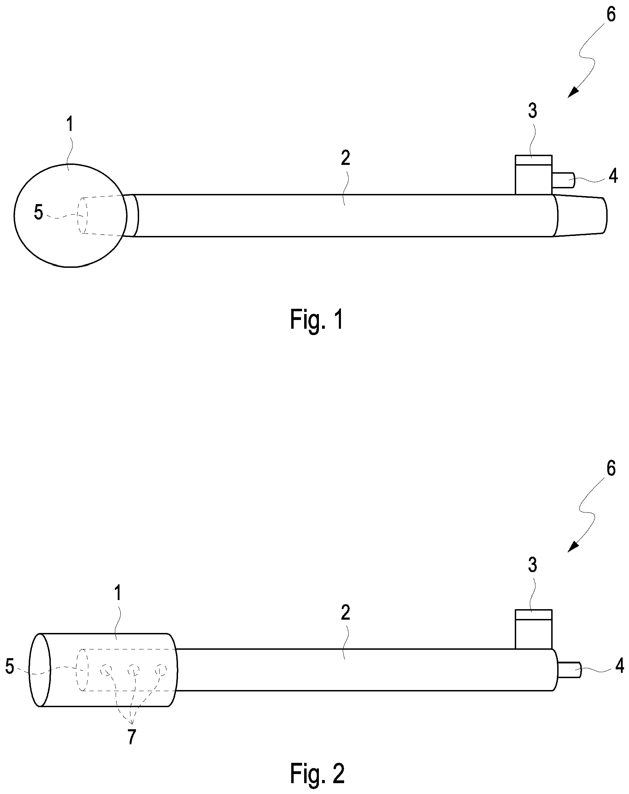

Fig. 1 zeigt eine Absaugvorrichtung mit einem Saugrohr 2, um die Öffnung der Absaugvorrichtung an eine Operationsstelle eines Menschen oder eines Tieres zu befördern. Mit einem Saugknopf 3 kann das Saugen ein- bzw. ausgeschaltet werden. Vorteilhafterweise weist die Absaugvorrichtung um die Öffnung 5 einen Schwamm 1 auf, wodurch Blut und Sekrete durch den Schwamm 1 zusätzlich aufgesaugt werden können. Vorteilhafterweise werden durch das Absaugen durch die Öffnung 5 Blut und Sekrete der Operationsöffnung in den Schwamm 1 befördert, wodurch das Blut und die Sekrete aus der Operationsöffnung entfernt werden können.

Fig. 2 zeigt eine alternative Ausführungsform der Erfindung, wobei die Absaugvorrichtung 6 in der Nähe der vorderen Öffnung 5 weitere Öffnungen 7 zum Absaugen von Blut und Sekreten aufweisen kann. Diese zusätzlichen Absaugöffnungen sind seitlich an der Absaugvorrichtung 6 angeordnet. Vorteilhafterweise sind sämtliche Öffnungen 5 und 7, die eine Absaugwirkung entfalten, von einem Schwamm umgeben, sodass sich angesaugtes Blut und angesaugte Sekrete in dem Schwamm 1 verfangen können und aus der Operationswunde entfernt werden können.

Durch die Anordnung des Schwamms 1 wird insbesondere ein Trockenlegen der Operationswunde ermöglicht, wodurch der chirurgische Eingriff einfacher durchgeführt werden kann, da Blut und Sekrete nicht die Sicht auf die Operationswunde verhindern.

Es sei angemerkt, dass der Begriff "umfassen" weitere Elemente oder Verfahrensschritte nicht ausschließt, ebenso wie der Begriff "ein" und "eine" mehrere Elemente und Schritte nicht ausschließt.

Die verwendeten Bezugszeichen dienen lediglich zur Erhöhung der Verständlichkeit und sollen keinesfalls als einschränkend betrachtet werden, wobei der Schutzbereich der Erfindung durch die Ansprüche wiedergegeben wird.

## Patentansprüche

1. Absaugvorrichtung (6) zur Durchführung eines chirurgischen Eingriffs bei einem Menschen oder einem Tier, umfassend:
ein Saugrohr (2), wobei das Saugrohr (2) zum Abführen von angesaugtem Blut und angesaugten Sekreten vorgesehen ist,
eine vordere Öffnung (5) des Saugrohrs (2) zur Aufnahme von angesaugtem Blut und angesaugten Sekreten,
**dadurch gekennzeichnet, dass**
ein Schwamm (1) vor der Öffnung (5) angeordnet ist.

2. Absaugvorrichtung (6) nach Anspruch 1, wobei die Absaugvorrichtung (6) eine seitliche Öffnung (7) zur Aufnahme von angesaugtem Blut und angesaugten Sekreten aufweist.

3. Absaugvorrichtung (6) nach Anspruch 2, wobei vor der seitlichen Öffnung (7) der Schwamm (1) angeordnet ist.
